# EUROPEAN PATENT SPECIFICATION

(11) **EP 0 321 158 B1**
(45) Date of publication and mention of the grant of the patent: **07.04.1993**
(21) Application number: 88311684.0
(22) Date of filing: 09.12.1988
(51) Int. Cl.: C07C 213/10, C07C 215/76

(54) **Production of m-aminophenol**
Herstellung von m-Aminophenol
Production de m-aminophénol

(30) Priority: 12.12.1987 JP 315038/87; 23.12.1987 JP 327764/87
(43) Date of publication of application: 21.06.1989
(73) Proprietor: MITSUI PETROCHEMICAL INDUSTRIES, LTD., Tokyo 100 (JP)
(72) Inventor: Mizuno, Kenichi, Iwakuni Yamaguchi (JP); Fujimoto, Tadaaki, Kuga-gun Yamaguchi (JP); Kondo, Masahiro, Iwakuni Yamaguchi (JP)
(74) Representative: Myerscough, Philip Boyd

(56) References cited:
- EP-A- 0 168 976
- EP-A- 0 224 625

## Description

This invention relates to production and separation of m-aminophenol, and more particularly to a method of purifying an aqueous solution of m-aminophenol which contains m-phenylenediamine as impurities therein, and to a process of producing m-aminophenol making use of the above method.

A process of producing m-aminophenol is known, in which resorcinol is reacted with ammonia to convert one of the hydroxyls of the resorcinol to an amino group, as described in Japanese Patent Publication Nos. 60-13026 and 60-16929, and Japanese Patent Laid-open No. 62-215358. However, since the reaction is usually accompanied by by-production of m-phenylenediamine, 3,3′-dihydroxydiphenylamine, and the like, it is necessary to remove such by-products to obtain high purity m-aminophenol. However, m-aminophenol has a boiling point close to that of m-phenylenediamine, but also forms maximum boiling point azeotropes with m-phenylenediamine, so that m-aminophenol cannot be separated by normal distillation methods.

Moreover, m-aminophenol and m-phenylenediamine are both aromatic primary amines and very similar in chemical properties to each other, so that it is also difficult to separate them from each other by chemical means such as extraction or oxidative decomposition.

Therefore, there has been proposed a method of separating m-aminophenol from m-phenylenediamine in which m-aminophenol containing therein m-phenylenediamine as impurities is dissolved in water, and then m-aminophenol is selectively crystallized from the solution since m-phenylenediamine is more soluble in water, as is disclosed in Japanese Patent Laid-open No. 61-7239. A further method is also known in which ethers are used in place of water, and m-aminophenol is likewise crystallized selectively from the solution, as is disclosed in Japanese Patent Laid-open No. 61-5059.

However, it is disadvantageous to adopt the above mentioned separation methods in the production of m-aminophenol by the process as before described since some portions of m-aminophenol remain in mother liquor and hence yields of m-aminophenol are small.

It is, therefore, an object of the invention to provide a method of purifying an aqueous solution of m-aminophenol which contains m-phenylenediamine as impurities, thereby to make it possible to recover m-aminophenol of high purity from the solution.

It is a further object of the invention to provide a process of producing very high purity m-aminophenol which contains substantially no m-phenylenediamine as impurities.

According to the invention, there is provided a method of purifying an aqueous solution of m-aminophenol containing m-phenylenediamine as impurities therein, which comprises: extracting the m-phenylenediamine from the aqueous solution with a halogenated hydrocarbon.

Further according to the invention, there is provided an improvement in a process of producing m-aminophenol which makes use of the above method. The improvement in the process of producing m-aminophenol comprising reacting resorcinol with ammonia in water in the presence of a water soluble amination catalyst at elevated temperatures in a reaction vessel, and then separating m-aminophenol from the resultant reaction mixture, the improvement comprising:
(a) a step of removing unreacted ammonia from the reaction mixture and then crystallizing crude m-aminophenol from the reaction mixture;
(b) a step of separating the crude m-aminophenol from the reaction mixture;
(c) a step of adding a halogenatated hydrocarbon to the resulting mother liquor to extract m-phenylenediamine as by-products into the halogenatated hydrocarbon, separating an aqueous phase from an organic phase, and recycling the aqueous phase to the reaction vessel; and
(d) a step of distilling the crude m-aminophenol to provide high purity m-aminophenol.

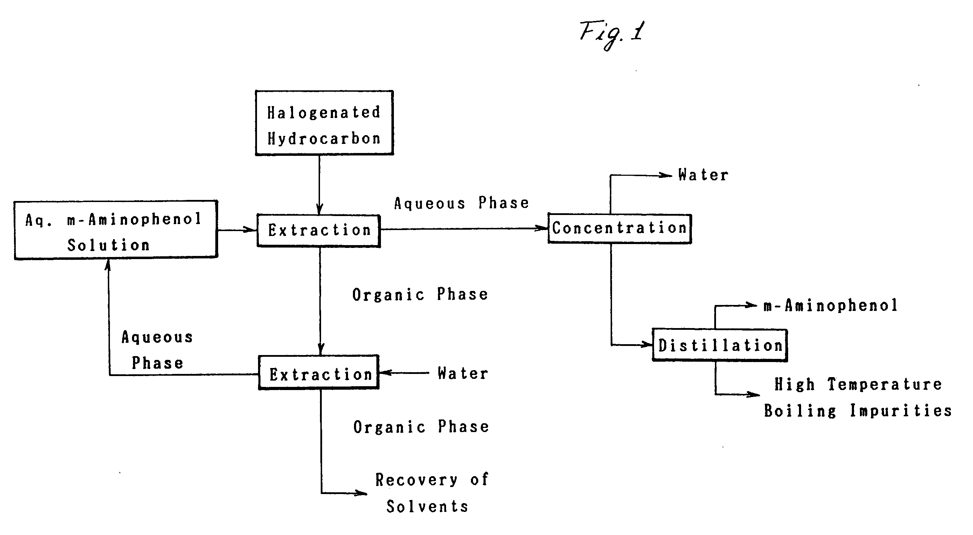

The method of purification of an aqueous solution of m-aminophenol containing m-phenylenediamine therein as impurities will be first described with reference to a flow chart of the method as illustrated in Fig. 1.

The method is suitably applicable to an aqueous solution which contains m-aminophenol in amounts of 5-70 %, preferably of 10-50 % by weight based on the solution, together with m-phenylenediamine in amounts of 0.5-20 %, preferably of 1-10 % by weight, based on the m-aminophenol. The solution may contain high boiling point substances such as 3,3′-dihydroxydiphenylamine, small amounts of resorcinol and catalysts, as may be the case when the method is applied to a mother liquor after the crystallization of m-aminophenol in the production of m-aminophenol as will be hereinafter described.

According to the method of the invention, a halogenated hydrocarbon is added to an aqueous solution of m-aminophenol containing m-phenylenediamine therein as impurities, to extract the m-phenylenediamine selectively therewith from the aqueous solution, while the m-aminophenol remains in the aqueous phase.

The halogenated hydrocarbon used as an extract includes halogenated saturated aliphatic hydrocarbons and halogenated aromatic hydrocarbons, preferably chlorinated ones. More specifically, the chlorinated saturated aliphatic hydrocarbons used are those having one or two carbons and two to five chlorines, and may be exemplified by methylene chloride, chloroform, 1,1-dichloroethane, 1,2-dichloroethane, 1,1,1-trichloroethane, 1,1,2-trichloroethane, pentachloroethane or dichloropropanes, with methylene chloride, chloroform or 1,2-dichloroethane most preferred. The chlorinated aromatic hydrocarbons used may be exemplified by chlorobenzene or o-dichlorobenzene. The chlorinated hydrocarbons may be used singly or as a mixture.

The amount of the halogenated hydrocarbon added to the aqueous solution depends upon the amount of the m-phenylenediamine contained in the solution. However, it is usually in the range of 0.1-20 parts by weight, preferably of 0.2-10 parts by weight, and most preferably of 1-5 parts by weight in relation to one part of the aqueous solution. In general, when the amount of the halogenated hydrocarbon is less than 0.1 part by weight per part of the aqueous solution, the m-phenylenediamine is insufficiently extracted into the halogenated hydrocarbon, whereas when the amount of the halogenated hydrocarbon is more than 20 parts by weight, much energy is needed for recovery of the halogenated hydrocarbon.

The extraction may be effected either in a batchwise or a continuous manner, but preferably in a continuous countercurrent manner, usually at 10-60°C, preferably at 25-40°C, using an extraction tower into which the aqueous solution is introduced from the bottom and the halogenated hydrocarbon from the top, for instance. Although not limited, an extraction tower used has preferably a number of theoretical plates of 2-10.

After the extraction, the resultant aqueous phase which contains m-aminophenol and the organic phase which contains the m-phenylenediamine are separated from each other. The removal of water from the aqueous phase by, for example, concentration, and distillation of the concentrate under reduced pressures provide high purity m-aminophenol while high boiling point temperature substances remain in the residual. The distillation is carried out usually at 120-300°C, preferably at 140-250°C under a pressure of 0.1-600 mmHg, preferably of 1-400 mmHg, either in a batchwise or a continuous manner.

The organic phase usually contains small amounts of m-aminophenol, and it is desired to recover the m-aminophenol from the organic phase. The recovery may be effected by various methods. For example, water is added to the orgaic phase to extract the m-aminophenol therewith, and the resultant aqueous phase is separated from the organic phase. The amount of water may be in the range of 0.1-10 parts by weight in relation to one part of the organic phase. The extraction may be carried out usually at 10-100°C, preferably at 30-80°C, preferably in a countercurrent manner as described hereinbefore. Then it is desired that the aqueous phase is combined with an aqueous m-aminophenol solution, and is again subjected to extraction with halogenated hydrocarbons. The halogenated hydrocarbons may be recovered and reused.

As a further method, formalin is added to the organic solution which contains m-aminophenol and m-phenylenediamine so that the latter selectively reacts with the formalin to produce precipitates. Then the precipitates are removed, and then the organic solvent is removed from the solution, to provide high purity m-aminophenol. As a still further method, the organic solution may be cooled so that m-aminophenol crystallizes out.

As set forth above, according to the method of the invention, an aqueous solution of m-aminophenol which contains m-phenylenediamine therein as impurities is extracted with a halogenated hydrocarbon, so that the resultant aqueos phase contains substantially no m-phenylenediamine, and thus the method makes it possible to obtain high purity m-aminophenol from the starting solution.

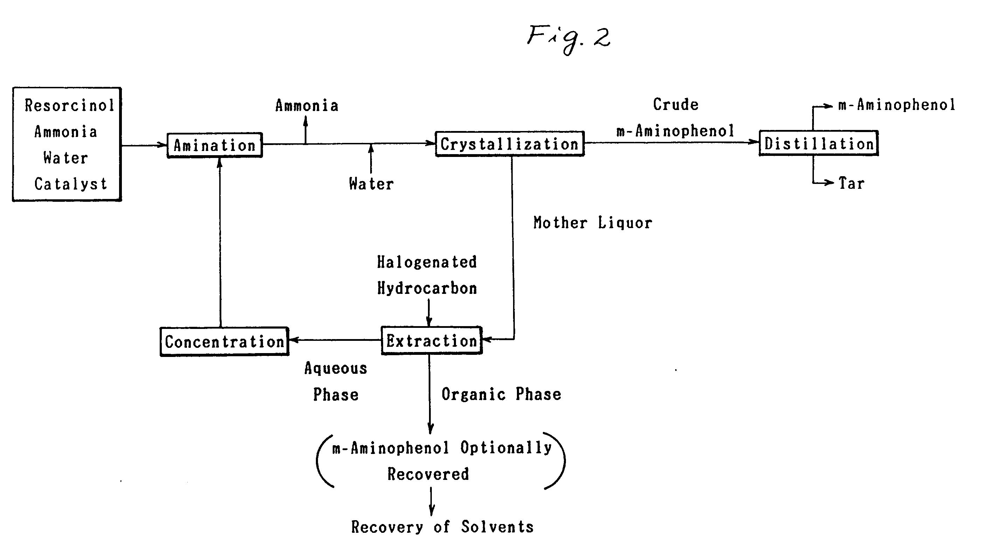

The production of m-aminophenol will now be described with reference to Fig. 2, a flow chart illustrating of the process. As shown therein, in the process of the invention, resorcinol is first reacted with ammonia in water in the presence of a water soluble aminaion catalyst.

The amination catalyst is already known, and a variety of metal compounds, ammonium salts, or compounds which produce ammonium compounds in the reaction conditions may be used as a catalyst. The amination catalyst used in the process of the invention includes, for example, metal compounds such as oxides, sulfates or ammonium salts of molybdenum, copper, antimony, vanadium, iron or nickel; and ammonium halides such as ammonium chloride, ammonium bromide or ammonium iodide. Other ammonium salts such as ammonium nitrate, ammonium sulfate, ammonium phosphate, heteropolyacid ammoniums, isopolyacid ammoniums such as ammonium molybdate or ammonium tungstate may also be used as a catalyst. Further, since acidic compounds such as hydrochloric acid, hydrobromic acid or, heteropolyacid or isopolyacid containing molybdenum or tungsten, produce ammonium compounds in the reaction conditions, they also may be used as a catalyst.

In the reaction, ammonia is used in amounts of not less than one mole, preferably of 1-5 moles, per mole of resorcinol, whereas water is used so that the ammonia dissolved therein has a concentration of 10-60 % by weight. The catalyst is used in amounts of 0.001-2 moles, preferably of 0.005-1 mole per mole of resorcinol.

The reaction of resorcinol with ammonia is carried out in a liquid phase under an atmosphere of an inert gas such as nitrogen usually at temperatures of 170-350°C, preferably of 180-300°C and at pressures of 5-150 kg/cm²G, preferably of 10-50 kg/cm²G. The reaction may be carried out either in batchwise or semicontinuous or continuous manners.

After completion of the reaction, a reaction mixture is obtained which contains therein, as by-products, m-phenylenediamine and other high boiling temperature compounds such as 3,3′-dihydroxydiphenylamine, in addition to m-aminophenol. The reaction mixture further contains unreacted resorcinol, ammonia and the catalyst.

When ammonia is used in amounts more than theoretical, it is advantageous to recover the unreacted ammonia from the reaction mixture for reuse. The unreacted ammonia may be usually distilled off from the reaction mixture, and is then dissolved in water to recover the ammonia as aqueous solutions. More specifically, after the reaction, the reaction mixture is maintained usually at temperatures of 80-250°C, preferably of 100-250°C while being reduced in pressures to 0-10 kg/cm²G, thereby to distill the ammonia from the reaction mixture, and then the ammonia is dissolved in water.

Then crude m-aminophenol is crystallized from the reaction mixture. In this crystallizing step (a), it is preferred that the concentration of m-aminophenol in the reaction mixture is in advance adjuted to 5-60 %, preferably of 10-45 % by weight. Usually warm water or, mother liquor or washing which has been produced in a previous crystallizing step may be added to the reaction mixture to adjust the m-aminophenol concentration therein. It may be also advantageous that the reaction mixture is heated in advance to 60-100°C to provide a uniform mixture. The reaction mixture is then cooled to temperatures of 0-50°C, preferably of 25-45°C usually at rates of 0.001-1°C/minute, preferably of 0.01-0.5°C/minute, thereby to crystallize crude m-aminophenol from the reaction mixture.

Then in the step (b), the crude m-aminophenol is separated from the reaction mixture by, for example, decantation, filtration or centrifuging, usually at temperatures of 0-60°C, preferably at temperatures of 10-45°C. m-Phenylenediamine as by-products is water soluble so that it remains in the mother liquor, and thus the separated m-aminophenol contains substantially no m-phenylenediamine.

The mother liquor still contains a small amount of m-aminophenol as well as the m-phenylenediamine therein. Therefore, in accordance with the invention, a halogenated hydrocarbon is added to the mother liquor to extract the m-phenylenediamine therewith, while the m-aminophenol remains in an aqueous phase. In this step (c), the halogenated hydrocarbon set out hereinbefore is used as an extract.

As also described hereinbefore, the amount of the halogenated hydrocarbon added to the mother liquor depends upon the amount of the m-phenylenediamine contained in the mother liquor, however it is usually in the range of 0.2-10 parts by weight, preferably of 1-5 parts by weight, in relation to one part of the mother liquor. The extraction is effected either in a batchwise or a continuous manner, but preferably in a continuous countercurrent manner, usually at 10-60°C, preferably at 25-40°C, using an extraction tower into which the mother liquor is introduced from the bottom and the halogenated hydrocarbon from the top, for instance.

After the extraction, the resultant aqueous phase which still contains a small amount of m-aminophenol is concentrated if desired, and then recycled to the reaction vessel, thereby to increase yields of m-aminophenol. m-Aminophenol may be recovered from the halogenated hydrocarbon phase if desired, by, for example, concentration of the phase, or by extraction with water, and then the organic phase may be distilled to recover the halogenated hydrocarbon for reuse.

On the other hand, the crude m-aminophenol obtained in the step (b) is subjected to distillation to remove tar materials as distillation residuals, thus to provide high purity m-aminophenol as distillates. The distillation of the crude m-aminophenol is effected usually at 120-250°C, preferably at 140-220°C, usually at 0.1-600 mmHg, preferably at 1-400 mmHg, either in a batchwise or a continuous manner.

As above described, since the process of the invention involves the steps (a) to (d), in particular the steps of extracting the by-produced m-phenylenediamine with a halogenated hydrocarbon from the reaction mixture after the crystallization of m-aminophenol therefrom, and of recycling the resultant mother liquor for reuse in the reaction, high purity m-aminophenol is obtained in high yields.

The invention will now be more fully described with reference to examples, however, the examples are illustrative only, and the invention is not limited thereto.

### Example 1

An aqueous solution containing m-aminophenol in amounts of 4.35 % by weight, m-phenylenediamine in amounts of 0.13 % by weight and high temperature boiling point substances such as 3,3′-dihydroxydiphenylamine in amounts of 0.27 % by weight was introduced as a feed solution into a countercurrent extraction tower having a number of theoretical plates of 8 from the bottom at a rate of 100 g/hr., while methylene chloride was supplied to the tower from the top at a rate of 180 g/hr. at 35°C, to carry out the extraction of the m-phenylenediamine at 35°C.

As a result, there were obtained a methylene chloride solution containing m-aminophenol in amounts of 0.25 % by weight and m-phenylenediamine in amounts of 0.066 % by weight from the bottom at a rate of 181 g/hr., and an aqueous solution containing m-aminophenol in amounts of 3.94 % by weight and m-phenylenediamine in amounts of 0.011 % by weight from the top at a rate of 99 g/hr.

It was found, therefore, that 91.9 % of the m-phenylenediamine contained in the feed solution were extracted with the methylene chloride, while 89.7 % of m-aminophenol remained in the aqueous solution.

Then the aqueous phase was concentrated to remove the water, and distilled under reduced pressures, to provide m-aminophenol in 99.7 % purity.

### Example 2

Except that chloroform was used as an extract in place of methylene chloride, and was introduced into the extraction tower at a rate of 350 g/hr., the extraction was carried out in the same manner as in Example 1. It was found that 85.3 % of the m-phenylenediamine contained in the feed solution were extracted with the chloroform, while 87.1 % of m-aminophenol remained in the aqueous solution.

### Example 3

(1) Crude m-aminophenol containing m-aminophenol in amounts of 88.7 % by weight, m-phenylenediamine in amounts of 3.67 % by weight and high temperature boiling point substances such as 3,3′-dihydroxydiphenylamine in amounts of 7.3 % by weight was dissolved at a temperature of 60°C at a rate of 86.9 g/hr. in an aqueous solution containing m-aminophenol in amounts of 9.6 % by weight and m-phenylenediamine in amounts of 0.48 % by weight supplied from a second extraction tower.
   The resultant solution was supplied to a first countercurrent extraction tower having a number of theoretical plates of 7 from the bottom at a rate of 100g/hr., while 1,2-dichloroethane at a temperature of 60°C was supplied to the tower from the top at a rate of 200 g/hr., to carry out the extraction at 60°C.
   As a result, there were obtained an aqueous solution containing m-aminophenol in amounts of 12.3 % by weight and m-phenylenediamine in amounts of 0.046 % by weight from the top of the first tower at a rate of 89.4 g/hr., and a 1,2-dichloroethane solution containing m-aminophenol in amounts of 4.27 % by weight and m-phenylenediamine in amounts of 0.408 % by weight from the bottom at a rate of 210 g/hr.
(2) The 1,2-dichloroethane solution from the bottom of the first extraction tower was supplied to the second countercurrent extraction tower having a number of theoretical plates of 5 from the top at a rate of 210 g/hr., while warm water at 60°C was supplied to the second tower from the bottom at a rate of 77.8 g/hr., to carry out the extraction at 60°C.
   As a result, there were obtained an aqueous solution containing m-aminophenol in amounts of 9.6 % by weight and m-phenylenediamine in amounts of 0.48 % by weight from the top of the second tower at a rate of 86.9 g/hr., and a 1,2-dichloroethane solution containing m-aminophenol in amounts of 0.31 % by weight and m-phenylenediamine in amounts of 0.22 % by weight from the bottom at a rate of 200.9 g/hr.
   The aqueous solution from the second extraction tower was used to dissolve the crude m-aminophenol as described in the step of (1).
(3) The aqueous solution obtained from the top of the first extraction tower in the step of (1) was distilled to remove the water, and then the concentrate was distilled under reduced pressures, to provide m-aminophenol in 99.6 % at a rate of 10.83 g/hr. It was, therefore, found that 93.5 % of the crude m-aminophenol were recovered as high purity m-aminophenol.

### Example 4

Except that 1,2-dichloropropane was used as an extract in place of methylene chloride, and was introduced into the extraction tower at a rate of 450 g/hr., the extraction was carried out in the same manneras as in Example 1. It was found as results that 93.6 % of the m-phenylenediamine contained in the feed solution were extracted with the 1,2-dichloropropane, while 82.3 % of m-aminophenol remained in the aqueous phase.

### Example 5

Except that chlorobenzene was used as an extract in place of methylene chloride, and was introduced into the extraction tower at a rate of 1500 g/hr., the extraction was carried out in the same manner as in Example 1, It was found as results that 92.1 % of the m-phenylenediamine contained in the feed solution were extracted with the chlorobenzene, while 80.7 % of m-aminophenol remained in the aqueous phase.

### Example 6

(1) In a SUS 316 reaction vessel were placed 1100 g of resolcinol, 350 g of ammonium molybdate ((NH₄)₇Mo₆O₂₄·4H₂O) and 1821 g of 28 % by weight ammonia water, and the reaction was carried out at 200°C over 8 hours under stirring.
   After completion of the reaction, the reaction mixture was cooled to 140°C and the pressure inside the vessel was decreased, to distill unreacted ammonia from the reaction mixture. The distilled ammonia was dissolved in water, to provide 839 g of a 40 % by weight aqueous ammonia.
(2) After the distillation of ammonia as above, the reaction mixture of 2420 g was cooled to 85°C, and then 4400 g of hot water at 85°C were added thereto. After fully stirring, the mixture was cooled to 35°C in 6 hours, to crystallize crude m-aminophenol. The crude m-aminophenol was collected by centrifuging at 35°C.
   The crude m-aminophenol thus separated was washed with 740 g of water, and the washings were combined with the mother liquor. As results, there were obtained 744 g of 90.5 % purity crude m-aminophenol and 6810 g of a mother liquor (containing the washings) containing m-aminophenol in amounts of 4.23 % by weight, resorcinol in amounts of 0.32 % by weight and m-phenylenediamine in amounts of 0.51 % by weight. Therefore, the conversion of the resorcinol was found 98.0 % and the selectivity of the reaction to m-aminophenol was found 90.0 %.
(3) The aforesaid mother liquor (containing the washings) was supplied to a countercurrent extraction tower having a number of theoretical plates of 7 from the bottom at a rate of 100 g/hr., while 1,2-dichloroethane was supplied to the tower from the top at a rate of 200 g/hr. at 30°C, to provide 6751 g of an aqueous solution from the top containing m-aminophenol in amounts of 3.46 % by weight, resorcinol in amounts of 0.32 % by weight and m-phenylenediamine in amounts of 0.04 % by weight. It was found, therefore, that 92 % of the m-phenylenediamine were extracted with the 1,2-dichloroethane from the mother liquor, while 81.1 % of m-aminophenol remained in the aqueous phase.
(4) Meanwhile, the crude m-aminophenol of 744 g obtained in the step (2) was distilled at 160°C and 7 mmHg, to provide 666 g of m-aminophenol in 99.56 % purity.
(5) The aforesaid aqueous solution of 6751 g obtained in the step (3) was concentrated to 1658 g, and then returned to the reaction vessel.

### Example 7

(1) In the reaction vessel to which the aqueous solution had been added in the step (5) of Example 1 were placed 812 g of resorcinol, 386 g of liquid ammonia and 3 g of ammonium molybdate, and the reaction was carried out at 200°C over 8 hours under stirring.
   After completion of the reaction, the reaction mixture was cooled to 140°C and the pressure inside the vessel was decreased, to distill unreacted ammonia from the reaction mixture. The distilled ammonia was then dissolved in water, to provide 618 g of a 41 % by weight aqueous ammonia.
(2) After the distillation of ammonia as above, the reaction mixture of 2232 g was cooled to 85°C, and then 4580 g of hot water at 85°C were added thereto. After fully stirring, the mixture was cooled to 35°C in 6 hours, to crystallize crude m-aminophenol. The crude m-aminophenol was collected by centrifuging at 35°C.
   The crude m-aminophenol thus separated was washed with 740 g of water, and the washings were combined with the mother liquor. As results, there were obtained 738 g of 91.1 % purity crude m-aminophenol and 6805 g of a mother liquor (containing the washings) containing m-aminophenol in amounts of 4.24 % by weight, resorcinol in amounts of 0.22 % by weight and m-phenylenediamine in amounts of 0.37 % by weight. Therefore, the conversion of the resorcinol in the reaction thereof with ammonia was found 98.2 % based on the resorcinol added anew to the reaction vessel, and the selectivity of the reaction to m-aminophenol was found 89.7 %.
(3) The crude m-aminophenol of 738 g obtained in the above step (2) was distilled at 160°C and 7 mmHg, to provide 668 g of m-aminophenol in 99.58 % purity.
(4) The aforesaid mother liquor (containing the washings) was supplied to a countercurrent extraction tower having a number of theoretical plates of 7 from the bottom at a rate of 100 g/hr., while 1,2-dichloroethane was supplied to the tower from the top at a rate of 200 g/hr. at 30°C, to provide 6748 g of an aqueous solution from the top. The aqueous solution was concentrated to 1649 g, and was then recycled to the reaction vessel.

### Reference Example 1

(1) After the steps (1) and (2) in Example 6, an amount of 6810 g of the mother liquor combined with the washings was concentrated to 1658 g under reduced pressures without extraction, and then was returned to the reaction vessel. An amount of 812 g of resorcinol, 386 g of liquid ammonia and 3 g of ammonium molybdate were then placed in the reaction vessel, and the reaction was carried out at 200°C over 8 hours under stirring.
   After completion of the reaction, the reaction mixture was cooled to 140°C and the pressure inside the vessel was decreased, to distill unreacted ammonia from the reaction mixture.
(2) After the distillation of ammonia as above, the reaction mixture of 2245 g was cooled to 85°C, and then 4820 g of hot water at 85°C were added thereto. After fully stirring, the mixture was cooled to 35°C in 6 hours, to crystallize crude m-aminophenol. The crude m-aminophenol was collected by centrifuging at 35°C.
   The crude m-aminophenol thus separated was washed with 740 g of water, and the washings were combined with the mother liquor. As results, there were obtained 794 g of 87.9 % purity crude m-aminophenol and 7011 g of a mother liquor (containing the washings) containing m-aminophenol in amounts of 4.25 % by weight, resorcinol in amounts of 0.25 % by weight and m-phenylenediamine in amounts of 0.52 % by weight. Therefore, the conversion of the resorcinol in the reaction thereof with ammonia was found 97.9 % based on the resorcinol added anew to the reaction vessel, and the selectivity of the reaction to m-aminophenol was found 87.5 %.
(3) The crude m-aminophenol of 794 g obtained in the above step (2) was distilled at 160°C and 7 mmHg, to provide 705 g of m-aminophenol. The m-aminophenol was found to contain m-phenylenediamine in amounts of 3.5 % by weight, and thus a low purity of 96.5 %.

## Claims

1. A method of purifying an aqueous solution of m-amino-phenol containing m-phenylenediamine as impurity, which comprises extracting the m-phenylenediamine from the aqueous solution with a halogenated hydrocarbon.

2. A method according to claim 1 wherein the halogenated hydrocarbon is a chlorinated saturated aliphatic hydrocarbon or chlorinated aromatic hydrocarbon.

3. A method according to claim 2 wherein there is used a chlorinated saturated aliphatic hydrocarbon having one or two carbons and two to five chlorines.

4. A method according to claim 3 wherein there is used at least one of methylene chloride, chloroform, 1,1-dichloroethane, 1,2-dichloroethane, 1,1,1-trichloroethane, 1,1,2-trichloroethane, pentachloroethane and a dichloropropane.

5. A method according to claim 2 wherein there is used as chlorinated aromatic hydrocarbon at least one of chlorobenzene and o-dichlorobenzene.

6. A process for producing m-aminophenol which comprises (i) reacting resorcinol with ammonia in water in the presence of a water soluble amination catalyst at elevated temperatures in a reaction vessel; (ii) removing unreacted ammonia from the reaction mixture and crystallizing crude m-aminophenol from the reaction mixture; (iii) separating a crude m-aminophenol from the reaction mixture to provide an aqueous mother liquor; (iv) extracting the mother liquor with a halogenated hydrocarbon as defined in any one of the preceding claims, separating an aqueous phase containing m-aminophenol from an organic phase containing m-phenylenediamine, and recycling the aqueous phase to the reaction vessel in step (i); and (v) distilling the crude m-aminophenol from step (ii) to provide high purity m-aminophenol.

## Patentansprüche

1. Verfahren zur Reinigung einer wäßrigen Lösung von m-Aminophenol, enthaltend m-Phenylendiamin als Verunreinigung, umfassend das Extrahieren des m-Phenylendiamins aus der wäßrigen Lösung mit einem halogenierten Kohlenwasserstoff.

2. Verfahren nach Anspruch 1, wobei der halogenierte Kohlenwasserstoff ein chlorierter gesättigter aliphatischer Kohlenwasserstoff oder ein chlorierter aromatischer Kohlenwasserstoff ist.

3. Verfahren nach Anspruch 2, wobei ein chlorierter gesättigter aliphatischer Kohlenwasserstoff mit ein oder zwei Kohlenstoffatomen und zwei bis fünf Chloratomen verwendet wird.

4. Verfahren nach Anspruch 3, wobei mindestens eine Verbindung, ausgewählt aus Methylenchlorid, Chloroform, 1,1-Dichlorethan, 1,2-Dichlorethan, 1,1,1-Trichlorethan, 1,1,2-Trichlorethan, Pentachlorethan und einem Dichlorpropan, verwendet wird.

5. Verfahren nach Anspruch 2, wobei als chlorierter aromatischer Kohlenwasserstoff Chlorbenzol und/oder o-Dichlorbenzol verwendet wird.

6. Verfahren zur Herstellung von m-Aminophenol, umfassend (i) die Umsetzung von Resorcin mit Ammoniak in Wasser in Gegenwart eines wasserlöslichen Aminierungskatalysators bei erhöhter Temperatur in einem Reaktionsgefäß, (ii) Entfernung von nicht umgesetztem Ammoniak aus dem Reaktionsgemisch und Auskristallisieren von rohem m-Aminophenol aus dem Reaktionsgemisch, (iii) Abtrennen eines rohen m-Aminophenols aus dem Reaktionsgemisch unter Bildung einer wäßrigen Mutterlauge, (iv) Extrahieren der Mutterlauge mit einem halogenierten Kohlenwasserstoff wie in einem der vorangehenden Ansprüche angegeben, Abtrennen einer wäßrigen Phase, enthaltend m-Aminophenol, von einer organischen Phase, enthaltend m-Phenylendiamin, und Zurückführen der wäßrigen Phase in das Reaktionsgefäß der Stufe (i) und (v) Destillieren des rohen m-Aminophenols von Stufe (ii) unter Bildung von m-Aminophenol mit hoher Reinheit.

## Revendications

1. Procédé de purification d'une solution aqueuse de m-aminophénol, renfermant de la m-phénylènediamine comme impureté, qui comprend l'extraction de la m-phénylènediamine à partir de la solution aqueuse, à l'aide d'un hydrocarbure halogéné.

2. Procédé selon la revendication 1, dans lequel l'hydrocarbure halogéné est un hydrocarbure aliphatique saturé chloré ou un hydrocarbure aromatique chloré.

3. Procédé selon la revendication 2, dans lequel on utilise un hydrocarbure aliphatique saturé chloré, ayant un ou deux atomes de carbone et deux à cinq atomes de chlore.

4. Procédé selon la revendication 3, dans lequel on utilise au moins un hydrocarbure chloré pris parmi le chlorure de méthylène, le chloroforme, le 1,1-dichloroéthane, le 1,2-dichloroéthane, le 1,1,1-trichloroéthane, le 1,1,2-trichloroéthane, le pentachloroéthane et les dichloropropanes.

5. Procédé selon la revendication 2, dans lequel on utilise, en tant qu'hydrocarbure aromatique chloré, au moins un composé pris parmi le chlorobenzène et l'orthodichlorobenzène.

6. Procédé de préparation de m-aminophénol, qui comprend (i) la réaction dans un récipient de réaction, à température élevée, en présence d'un catalyseur d'amination soluble dans l'eau, de résorcinol avec de l'ammoniac dans de l'eau, (ii) l'élimination de l'ammoniac qui n'a pas réagi du mélange réactionnel et la cristallisation de m-aminophénol brut à partir du mélange réactionnel, (iii) la séparation d'un m-aminophénol brut du mélange réactionnel de façon à obtenir une liqueur-mère aqueuse, (iv) la soumission de la liqueur-mère à une extraction réalisée à l'aide d'un hydrocarbure halogéné tel que défini dans l'une quelconque des revendications précédentes, la séparation d'une phase aqueuse renfermant du m-aminophénol d'une phase organique renfermant de la m-phénylènediamine, et le recyclage de la phase aqueuse dans le récipient de réaction de l'étape (i), et (v) la distillation du m-aminophénol brut provenant de l'étape (ii) de façon à obtenir un m-aminophénol de pureté élevée.
